# EUROPEAN PATENT APPLICATION

(11) **EP 4 682 250 A1**
(43) Date of publication of application: **21.01.2026**
(21) Application number: 24770824.1
(22) Date of filing: 11.03.2024
(51) Int. Cl.: C12N 5/10, C07K 5/09, C12N 5/078, C12N 15/86

(54) **PRODUCTION METHOD FOR INDUCED PLURIPOTENT STEM CELLS**

(30) Priority: 14.03.2023 JP 2023040005
(71) Applicant: Sekisui Chemical Co., Ltd., Osaka-shi, Osaka 530-8565 (JP)
(72) Inventor: YANAGISAWA, Teruhiko, Tsukuba-shi, Ibaraki 300-4292 (JP); NAKAMURA, Yuuta, Mishima-gun, Osaka 618-0021 (JP); HANEDA, Satoshi, Mishima-gun, Osaka 618-0021 (JP)
(74) Representative: Ter Meer Steinmeister & Partner
(86) International application number: PCT/JP2024/009228
(87) International publication number: WO 2024/190718

(57) **Abstract**

Provided is a production method for induced pluripotent stem cells by which induced pluripotent stem cells having favorable quality can be easily obtained in a short period of time. A production method for induced pluripotent stem cells according to the present invention includes, in the stated order: a seeding step of seeding somatic cells on a cell culture substrate including a cell scaffold; a removal step of removing suspended somatic cells without adhering to the cell scaffold; and an introducing step of introducing a reprogramming factor into somatic cells adhering to the cell scaffold.

## Description

### TECHNICAL FIELD

The present invention relates to a production method for induced pluripotent stem cells.

### BACKGROUND ART

In research fields such as academic fields, drug discovery fields, and regenerative medicine fields, induced pluripotent stem cells (iPS cells) have attracted attention. The iPS cells can be produced by introducing a reprogramming factor (for example, OCT3/4, SOX2, KLF4, or c-MYC) into somatic cells (for example, Patent Document 1).

As a preparation method for iPS cells, a method of culturing somatic cells into which a reprogramming factor has been introduced on a laminin-coated plate is widely used (for example, Non-Patent Document 1). Laminin plays a role as a cell scaffold.

Meanwhile, Patent Document 2 below describes a scaffold material for cell culture, containing a peptide-conjugated polyvinyl alcohol derivative having a polyvinyl alcohol derivative moiety and a peptide moiety. In Examples of Patent Document 2, it is described that iPS cells were cultured using the scaffold material. However, Patent Document 2 does not describe establishment of iPS cells from somatic cells.

### Related Art Document

### Patent Documents

Patent Document 1: JP 2019-162054 A
Patent Document 2: WO 2020/230885 A1

### Non-Patent Document

Non-Patent Document 1: Protocol Human iPS cell culture under feeder-free conditions, CiRA_Ff-iPSC_protocol_JP_v140310 (Center for iPS Cell Research and Application, Kyoto University, https://www.cira.kyoto-u.ac.jp/j/research/img/protocol/hipsprotocolFf_140311.pdf, URL for English version: https://www.cira.kyoto-u.ac.jp/j/research/img/protocol/Ff-iPSC-culture_protocol_E_v140311.pdf)

### SUMMARY OF THE INVENTION

### PROBLEMS TO BE SOLVED BY THE INVENTION

Conventionally, induced pluripotent stem cells (iPS cells) are produced by seeding somatic cells on a cell culture container and culturing the somatic cells, then recovering suspended somatic cells in another container, and introducing a reprogramming factor into the recovered somatic cells. However, the conventional method requires a step of recovering suspended somatic cells in another container, and thus the operation is complicated.

It is difficult to obtain induced pluripotent stem cells having favorable quality in a short period of time by the conventional method. For example, the conventional method may not sufficiently increase the positive rate of TRA-1-60 which is an undifferentiated marker.

An object of the present invention is to provide a production method for induced pluripotent stem cells by which induced pluripotent stem cells having favorable quality can be easily obtained in a short period of time.

### MEANS FOR SOLVING THE PROBLEMS

In the present specification, the following production method for induced pluripotent stem cells is disclosed.

Item 1. A production method for induced pluripotent stem cells, including, in the stated order: a seeding step of seeding somatic cells on a cell culture substrate including a cell scaffold; a removal step of removing suspended somatic cells without adhering to the cell scaffold; and an introducing step of introducing a reprogramming factor into somatic cells adhering to the cell scaffold.

Item 2. The production method for induced pluripotent stem cells according to item 1, wherein the somatic cells seeded in the seeding step include peripheral blood mononuclear cells.

Item 3. The production method for induced pluripotent stem cells according to item 1 or 2, wherein the somatic cells adhering to the cell scaffold in the introducing step include cells having phagocytosis.

Item 4. The production method for induced pluripotent stem cells according to any one of items 1 to 3, wherein the cell scaffold has an RGD sequence.

Item 5. The production method for induced pluripotent stem cells according to any one of items 1 to 4, wherein the cell scaffold contains a peptide-conjugated resin having a synthetic resin moiety and a peptide moiety.

Item 6. The production method for induced pluripotent stem cells according to item 5, wherein the peptide-conjugated resin has a polyvinyl acetal resin moiety and a peptide moiety.

Item 7. The production method for induced pluripotent stem cells according to any one of items 1 to 6, wherein in the introducing step, a reprogramming factor is introduced into somatic cells adhering to the cell scaffold using a sendaiviral vector.

### EFFECT OF THE INVENTION

A production method for induced pluripotent stem cells according to the present invention includes, in the stated order: a seeding step of seeding somatic cells on a cell culture substrate including a cell scaffold; a removal step of removing suspended somatic cells without adhering to the cell scaffold; and an introducing step of introducing a reprogramming factor into somatic cells adhering to the cell scaffold. In the production method for induced pluripotent stem cells according to the present invention, since the above configuration is included, induced pluripotent stem cells having favorable quality can be easily obtained in a short period of time.

### BRIEF DESCRIPTION OF DRAWINGS

[Fig. 1] Fig.1 is a dot plot view of FSC-SSC when somatic cells removed by a removal step (suspended somatic cells) and somatic cells adhering to a cell scaffold after the removal step are each measured by flow cytometry in Example 1.
[Fig. 2] Fig. 2 is a micrograph when the phagocytosis of the somatic cells adhering to a cell scaffold after the removal step is evaluated in Example 1.
[Fig. 3] Fig. 3 is a micrograph of cells on Day 1 after somatic cells into which a reprogramming factor has been introduced are cultured in Example 1 and Comparative Example 2.
[Fig. 4] Fig. 4 is a view showing a relationship between the number of passages after somatic cells into which a reprogramming factor has been introduced are cultured and the residual rate of virus.
[Fig. 5] Fig. 5 is a view showing a relationship between the number of passages after somatic cells into which a reprogramming factor has been introduced are cultured and the expression level of TRA-1-60.
[Fig. 6] Fig. 6 is a micrograph of a colony of iPS cells established from monocytes in Example 3.

### MODES FOR CARRYING OUT THE INVENTION

Hereinafter, details of the present invention will be described.

A production method for induced pluripotent stem cells according to the present invention includes, in the stated order: a seeding step of seeding somatic cells on a cell culture substrate including a cell scaffold; a removal step of removing suspended somatic cells without adhering to the cell scaffold; and an introducing step of introducing a reprogramming factor into somatic cells adhering to the cell scaffold.

In the production method for induced pluripotent stem cells according to the present invention, since the above configuration is included, induced pluripotent stem cells having favorable quality can be easily obtained in a short period of time.

Hereinafter, in the present specification, the "induced pluripotent stem cells" may be referred to as "iPS cells".

Conventionally, iPS cells are produced by seeding somatic cells on a cell culture container and culturing the somatic cells, then recovering suspended somatic cells in another container, and introducing a reprogramming factor into the recovered somatic cells. The present inventors have surprisingly found that, when a reprogramming factor is introduced into not suspended somatic cells but somatic cells adhering to a cell scaffold after removing suspended somatic cells, iPS cells having favorable quality can be easily obtained in a short period of time. In the production method for iPS cells according to the present invention, since the steps from the seeding step to the introducing step can be performed using the same cell culture substrate without performing a complicated and careful operation of recovering suspended somatic cells, iPS cells can be easily produced in a simple process. In the production method for iPS cells according to the present invention, iPS cells having favorable quality can be obtained in a short period of time. For example, in the production method for iPS cells according to the present invention, iPS cells having a high expression level of TRA-1-60, which is an undifferentiated marker, can be obtained at an early stage. Therefore, in the production method for iPS cells according to the present invention, iPS cells having a high undifferentiation property can be easily established in a short period of time. In the production method for iPS cells according to the present invention, in the case of using a viral vector as a reprogramming factor, the time until disappearance of the viral vector is shortened, and iPS cells having a small residual amount of the viral vector can be obtained.

In the production method for iPS cells according to the present invention, as compared with the conventional method, the use amount of the reprogramming factor required for producing iPS cells can be reduced. Therefore, the production cost of iPS cells can also be reduced.

First, somatic cells and a cell culture substrate that can be used in the present invention will be described.

### (Somatic cells)

As the somatic cells, conventionally known somatic cells used for establishing iPS cells can be used. The somatic cells are preferably animal cells, more preferably mammalian cells, and still more preferably human cells.

Examples of the somatic cells include blood cells, fibroblasts, hepatocytes, pancreatic cells, intestinal epithelial cells, smooth muscle cells, and dental pulp cells. Examples of the origin of the somatic cells include peripheral blood, cord blood, skin tissue, and teeth.

Examples of the blood cells include nucleated cells such as mononuclear cells, neutrophils, eosinophils, basophils, and lymphocytes. The blood cells may be vascular endothelial progenitor cells, blood stem/progenitor cells, T cells, or B cells.

The somatic cells seeded in the seeding step are preferably blood cells, more preferably include mononuclear cells, and still more preferably include peripheral blood mononuclear cells (PMBC). In the seeding step, it is preferable to seed a cell population including peripheral blood mononuclear cells (PMBC) on a cell culture substrate including a cell scaffold. Peripheral blood mononuclear cells (PMBC) are particularly suitably used because they have low invasiveness to a patient at the time of collection, have a low possibility of genomic mutation, and are also easy to perform aseptic manipulation.

### (Cell culture substrate)

The cell culture substrate includes a cell scaffold. The cell culture substrate may be a cell culture container, a cell culture microcarrier, a cell culture fiber, or any other substrate.

The cell culture substrate preferably includes a substrate (cell culture substrate body) and a cell scaffold.

### <Substrate (cell culture substrate body)>

The material, size, and shape of the substrate are not particularly limited. As the substrate, a conventionally known substrate can be used.

Examples of the substrate include a container, a fiber, a nonwoven fabric, a hollow fiber, a particle, a film, and a porous membrane.

Examples of the material of the substrate include synthetic resins, metals, and glass. Examples of the synthetic resin include polystyrene, polyethylene, polypropylene, polyethersulfone, polycarbonate, polyester, polyisoprene, a cycloolefin polymer, polyimide, polyamide, polyamideimide, a (meth)acrylic resin, an epoxy resin, and silicone.

The substrate is preferably a container. The cell culture substrate is preferably a cell culture container. The cell culture substrate is preferably a cell culture container including a container (cell culture container body) and a cell scaffold disposed on a first surface of the container. In this case, the cell scaffold may be disposed on the entire first surface of the container, or may be disposed on a part of the first surface of the container.

The shape and size of the container (cell culture container body) are not particularly limited. Examples of the container (cell culture container body) include a 2 to 384-well plate, a single-layer flask, a multi-layer flask, a multi-plane flask, a dish, a roller bottle, a bag, an insert cup, and a microchannel chip. The container may be a closed-system container. The first surface of the container is preferably a bottom surface of the container.

The substrate is preferably a particle (base particle). The cell culture substrate is preferably a cell culture microcarrier. The cell culture substrate is preferably a cell culture microcarrier including a base particle and a cell scaffold covering an outer surface of the base particle. In this case, the cell scaffold may cover the entire outer surface of the base particle, or may cover a part of the outer surface of the base particle.

### <Cell scaffold>

The cell culture substrate includes a cell scaffold. The cell scaffold is used as a scaffold for cells when the cells are cultured.

Examples of the component contained in the cell scaffold include a cell adhesive protein and a synthetic resin. Only one kind of the component may be used, or two or more kinds thereof may be used in combination.

The cell scaffold preferably has an RGD sequence. More specifically, the cell scaffold preferably contains a component having an RGD sequence. In this case, the effects of the present invention can be more effectively exhibited.

Examples of the cell adhesive protein include laminin, vitronectin, and collagen. The component contained in the cell scaffold may be a component containing a cell adhesive protein such as Matrigel. Examples of the Matrigel include Matrigel derived from mouse sarcoma.

As described later, the synthetic resin may include a synthetic resin having a synthetic resin moiety and a peptide moiety (synthetic resin to which a peptide is bonded). The synthetic resin may include a synthetic resin having no peptide moiety (synthetic resin to which no peptide is bonded). Examples of the synthetic resin include a polyvinyl alcohol derivative, a peptide-conjugated polyvinyl alcohol derivative having a polyvinyl alcohol derivative moiety and a peptide moiety, a (meth)acrylic copolymer, and a peptide-conjugated (meth)acrylic copolymer having a poly(meth)acrylic acid ester moiety and a peptide moiety. Examples of the polyvinyl alcohol derivative include a polyvinyl acetal resin.

Hereinafter, in the present specification, the "synthetic resin having a synthetic resin moiety and a peptide moiety" may be referred to as "peptide-conjugated resin".

The cell scaffold preferably contains a cell adhesive protein or a synthetic resin, more preferably contains laminin, vitronectin, or a peptide-conjugated resin, and still more preferably contains a peptide-conjugated resin. In this case, the effects of the present invention can be more effectively exhibited.

### <Peptide-conjugated resin>

From the viewpoint of more effectively exhibiting the effects of the present invention and the viewpoint of enhancing the production efficiency of the cell culture substrate, the cell scaffold preferably contains a peptide-conjugated resin having a synthetic resin moiety and a peptide moiety. The peptide-conjugated resin is a synthetic resin to which a peptide is bonded. The peptide-conjugated resin has a synthetic resin moiety and a peptide moiety. Only one kind of the peptide-conjugated resin may be used, or two or more kinds thereof may be used in combination.

The synthetic resin moiety preferably has a polyvinyl alcohol derivative moiety or a poly(meth)acrylic acid ester moiety, and is more preferably a polyvinyl alcohol derivative moiety or a poly(meth)acrylic acid ester moiety. The peptide-conjugated resin preferably has a polyvinyl alcohol derivative moiety or a poly(meth)acrylic acid ester moiety and a peptide moiety. In this case, the effects of the present invention can be more effectively exhibited. The peptide-conjugated resin may have both a polyvinyl alcohol derivative moiety and a poly (meth) acrylic acid ester moiety.

In the peptide-conjugated resin having a polyvinyl alcohol derivative moiety, it is preferable that the polyvinyl alcohol derivative moiety and the peptide moiety are bonded to each other via a linker moiety. Therefore, the peptide-conjugated resin having a polyvinyl alcohol derivative moiety preferably has a polyvinyl alcohol derivative moiety, a peptide moiety, and a linker moiety.

In the peptide-conjugated resin having a poly(meth)acrylic acid ester moiety, the poly(meth)acrylic acid ester moiety and the peptide moiety may be bonded to each other via a linker moiety, or may be bonded directly to each other without a linker moiety. The peptide-conjugated resin having a poly(meth) acrylic acid ester moiety may have a poly(meth) acrylic acid ester moiety, a peptide moiety, and a linker moiety.

In the present specification, "(meth) acrylic" means one or both of "acrylic" and "methacrylic", and "(meth)acrylate" means one or both of "acrylate" and "methacrylate".

### <<Polyvinyl alcohol derivative moiety>>

The polyvinyl alcohol derivative moiety (polyvinyl alcohol derivative skeleton) is a structural moiety derived from a polyvinyl alcohol derivative. The polyvinyl alcohol derivative is a compound derived from polyvinyl alcohol.

The peptide-conjugated resin is preferably a peptide-conjugated polyvinyl alcohol derivative having a polyvinyl alcohol derivative moiety and a peptide moiety. The cell scaffold preferably contains a peptide-conjugated polyvinyl alcohol derivative having a polyvinyl alcohol derivative moiety and a peptide moiety. By using the peptide-conjugated polyvinyl alcohol derivative, the effects of the present invention can be more effectively exhibited, and the cell proliferation can be further enhanced.

From the viewpoint of further effectively exhibiting the effects of the present invention and the viewpoint of further enhancing the cell proliferation, the polyvinyl alcohol derivative is preferably a polyvinyl acetal resin, and the polyvinyl alcohol derivative moiety is preferably a polyvinyl acetal resin moiety. That is, the peptide-conjugated resin preferably has a polyvinyl acetal resin moiety and a peptide moiety. The cell scaffold preferably contains a peptide-conjugated polyvinyl acetal resin having a polyvinyl acetal resin moiety and a peptide moiety. Only one kind of each of the polyvinyl alcohol derivative and the polyvinyl acetal resin may be used, or two or more kinds thereof may be used in combination.

The polyvinyl alcohol derivative moiety and the polyvinyl acetal resin moiety preferably have an acetal group, a hydroxyl group, and an acetyl group in side chains. However, the polyvinyl alcohol derivative moiety and the polyvinyl acetal resin moiety may not have, for example, an acetyl group. For example, all of the acetyl groups of the polyvinyl acetal resin moiety are bonded to a linker, so that the polyvinyl alcohol derivative moiety and the polyvinyl acetal resin moiety may not have an acetyl group.

The polyvinyl acetal resin can be synthesized by acetalizing polyvinyl alcohol with an aldehyde.

The aldehyde used for acetalization of polyvinyl alcohol is not particularly limited. Examples of the aldehyde include an aldehyde having 1 to 10 carbon atoms. The aldehyde may or may not have a chain aliphatic group, a cyclic aliphatic group, or an aromatic group. The aldehyde may be a chain aldehyde or a cyclic aldehyde. Only one kind of the aldehyde may be used, or two or more kinds thereof may be used in combination.

From the viewpoint of further enhancing the adhesiveness between the cell scaffold and the cell and the viewpoint of more effectively exhibiting the effects of the present invention, the aldehyde is preferably formaldehyde, acetaldehyde, propionaldehyde, butyraldehyde, or pentanal, and more preferably butyraldehyde. Therefore, the polyvinyl acetal resin is more preferably a polyvinyl butyral resin. From the viewpoint of further enhancing the adhesiveness between the cell scaffold and the cell and the viewpoint of more effectively exhibiting the effects of the present invention, the polyvinyl acetal resin moiety is preferably a polyvinyl butyral resin moiety. The peptide-conjugated resin preferably has a polyvinyl butyral resin moiety and a peptide moiety. The cell scaffold preferably contains a peptide-conjugated polyvinyl butyral resin having a polyvinyl butyral resin moiety and a peptide moiety.

In the peptide-conjugated polyvinyl acetal resin, the acetalization degree of the polyvinyl acetal resin moiety (butyralization degree in the case of the polyvinyl butyral resin moiety) is preferably 40 mol% or more and more preferably 50 mol% or more, and is preferably 90 mol% or less and more preferably 85 mol% or less. When the acetalization degree is the lower limit or more, the peptide-conjugated polyvinyl acetal resin is less likely to swell in the medium. When the acetalization degree is the upper limit or less, solubility in a solvent can be improved.

In the peptide-conjugated polyvinyl acetal resin, the content ratio of the hydroxyl group (hydroxyl group amount) of the polyvinyl acetal resin moiety is preferably 15 mol% or more and more preferably 20 mol% or more, and is preferably 45 mol% or less, more preferably 30 mol% or less, and still more preferably 25 mol% or less.

In the peptide-conjugated polyvinyl acetal resin, the acetylation degree (acetyl group amount) of the polyvinyl acetal resin moiety is preferably 1 mol% or more and more preferably 2 mol% or more, and is preferably 5 mol% or less and more preferably 4 mol% or less. When the acetylation degree is the lower limit or more and the upper limit or less, the reaction efficiency between the polyvinyl acetal resin and a linker can be enhanced.

The acetalization degree, the acetylation degree, and the hydroxyl group amount of the polyvinyl acetal resin moiety can be measured by ¹H-NMR (nuclear magnetic resonance spectrum).

### <<Poly(meth)acrylic acid ester moiety>>

The poly(meth)acrylic acid ester moiety (poly(meth)acrylic acid ester skeleton) is a structural moiety derived from a poly(meth)acrylic acid ester.

The peptide-conjugated resin preferably has a poly(meth)acrylic acid ester moiety, and is more preferably a peptide-conjugated (meth)acrylic copolymer having a poly(meth)acrylic acid ester moiety and a peptide moiety. The cell scaffold preferably contains a peptide-conjugated (meth)acrylic copolymer having a poly(meth)acrylic acid ester moiety and a peptide moiety. By using the peptide-conjugated (meth)acrylic copolymer, the effects of the present invention can be more effectively exhibited, and the cell proliferation can be further enhanced.

The poly(meth)acrylic acid ester moiety has a skeleton derived from a (meth)acrylic acid ester. The poly(meth)acrylic acid ester is obtained by polymerizing a (meth)acrylic acid ester. Only one kind of the poly (meth) acrylic acid ester may be used, or two or more kinds thereof may be used in combination.

Examples of the (meth)acrylic acid ester include a (meth)acrylic acid alkyl ester, a (meth)acrylic acid cyclic alkyl ester, a (meth)acrylic acid aryl ester, (meth)acrylic acid polyethylene glycols, and (meth)acrylic acid phosphorylcholine. Only one kind of the (meth)acrylic acid ester may be used, or two or more kinds thereof may be used in combination.

Examples of the (meth)acrylic acid alkyl ester include methyl (meth)acrylate, ethyl (meth)acrylate, n-propyl (meth)acrylate, isopropyl (meth)acrylate, n-butyl (meth)acrylate, isobutyl (meth)acrylate, t-butyl (meth)acrylate, n-octyl (meth)acrylate, isooctyl (meth)acrylate, 2-ethylhexyl (meth)acrylate, nonyl (meth)acrylate, isononyl (meth)acrylate, decyl (meth)acrylate, isodecyl (meth)acrylate, lauryl (meth)acrylate, stearyl (meth)acrylate, and isotetradecyl (meth) acrylate.

The (meth)acrylic acid alkyl ester may be substituted with a substituent such as an alkoxy group having 1 to 3 carbon atoms and a tetrahydrofurfuryl group. Examples of such a (meth)acrylic acid alkyl ester include methoxyethyl acrylate and tetrahydrofurfuryl acrylate.

Examples of the (meth)acrylic acid cyclic alkyl ester include cyclohexyl (meth)acrylate and isobornyl (meth)acrylate.

Examples of the (meth)acrylic acid aryl ester include phenyl (meth)acrylate and benzyl (meth)acrylate.

Examples of the (meth)acrylic acid polyethylene glycols include methoxy-polyethylene glycol (meth)acrylate, ethoxy-polyethylene glycol (meth)acrylate, hydroxy-polyethylene glycol (meth)acrylate, methoxy-diethylene glycol (meth)acrylate, ethoxy-diethylene glycol (meth) acrylate, hydroxy-diethylene glycol (meth)acrylate, methoxy-triethylene glycol (meth)acrylate, ethoxy-triethylene glycol (meth)acrylate, and hydroxy-triethylene glycol (meth)acrylate.

Examples of the (meth)acrylic acid phosphorylcholine include 2-(meth)acryloyloxyethyl phosphorylcholine.

The peptide-conjugated resin having a poly (meth) acrylic acid ester moiety may have a skeleton derived from a monomer other than the (meth)acrylic acid ester.

Examples of the monomer other than the (meth)acrylic acid ester include (meth)acrylamides and a vinyl compound. Only one kind of the monomer other than the (meth)acrylic acid ester may be used, or two or more kinds thereof may be used in combination.

Examples of the (meth)acrylamides include (meth)acrylamide, N-isopropyl (meth)acrylamide, N-tert-butyl (meth)acrylamide, N,N'-dimethyl (meth)acrylamide, (3-(meth)acrylamidepropyl)trimethylammonium chloride, 4-(meth)acryloylmorpholine, 3-(meth)acryloyl-2-oxazolidinone, N-[3-(dimethylamino)propyl] (meth)acrylamide, N-(2-hydroxyethyl) (meth)acrylamide, N-methylol (meth)acrylamide, and 6-(meth)acrylamidohexanoic acid.

Examples of the vinyl compound include ethylene, allylamine, vinylpyrrolidone, maleic anhydride, maleimide, itaconic acid, (meth)acrylic acid, and vinylamine.

### <<Peptide moiety>>

The peptide moiety (peptide skeleton) is a structural moiety derived from a peptide. The peptide moiety has an amino acid sequence. The peptide constituting the peptide moiety may be an oligopeptide or a polypeptide. Only one kind of the peptide may be used, or two or more kinds thereof may be used in combination.

The number of amino acid residues in the peptide moiety is preferably 3 or more, more preferably 4 or more, and still more preferably 5 or more, and is preferably 10 or less, more preferably 8 or less, and still more preferably 6 or less. When the number of amino acid residues is the lower limit or more and the upper limit or less, the adhesiveness between the cell scaffold and cells after seeding can be further enhanced, and the proliferation rate of cells can be further enhanced. However, the number of amino acid residues in the peptide moiety may be more than 10 or more than 15.

The peptide moiety preferably has a cell adhesive amino acid sequence. The cell adhesive amino acid sequence refers to an amino acid sequence whose cell adhesion activity has been confirmed by a phage display method, a sepharose bead method, or a plate coating method. As the phage display method, for example, the method described in "The Journal of Cell Biology, Volume 130, Number 5, September 1995 1189-1196" can be used. As the sepharose bead method, for example, the method described in "PROTEIN, NUCLEIC ACID, AND ENZYME, Vol. 45, No. 15 (2000) 2477" can be used. As the plate coating method, for example, the method described in "PROTEIN, NUCLEIC ACID, AND ENZYME, Vol. 45, No. 15 (2000) 2477" can be used.

Examples of the cell adhesive amino acid sequence include an RGD sequence (Arg-Gly-Asp), a YIGSR sequence (Tyr-Ile-Gly-Ser-Arg), a PDSGR sequence (Pro-Asp-Ser-Gly-Arg), an HAV sequence (His-Ala-Val), an ADT sequence (Ala-Asp-Thr), a QAV sequence (Gln-Ala-Val), an LDV sequence (Leu-Asp-Val), an IDS sequence (Ile-Asp-Ser), an REDV sequence (Arg-Glu-Asp-Val), an IDAPS sequence (Ile-Asp-Ala-Pro-Ser), a KQAGDV sequence (Lys-Gln-Ala-Gly-Asp-Val), and a TDE sequence (Thr-Asp-Glu). Examples of the cell adhesive amino acid sequence include the sequences described in "Pathophysiology, Vol. 9, No. 7, p. 527 to 535, 1990" and "Journal of Osaka Women's and Children's Hospital, Vol. 8, No. 1, p. 58 to 66, 1992". The peptide moiety may have only one type of the cell adhesive amino acid sequence or two or more types thereof.

The peptide moiety preferably has at least one of the above-described cell adhesive amino acid sequences, more preferably has at least an RGD sequence, a YIGSR sequence, or a PDSGR sequence, still more preferably has an RGD sequence, and particularly preferably has at least an RGD sequence represented by the following Formula (1). In this case, the adhesiveness between the cell scaffold and cells after seeding can be further enhanced, and the proliferation rate of cells can be further enhanced.

Arg-Gly-Asp-X Formula (1)

In the above Formula (1), X represents Gly, Ala, Val, Ser, Thr, Phe, Met, Pro, or Asn.

The peptide moiety may be linear or may have a cyclic peptide skeleton. The cyclic peptide skeleton is a cyclic skeleton composed of a plurality of amino acids. From the viewpoint of further effectively exhibiting the effects of the present invention, the cyclic peptide skeleton is preferably composed of four or more amino acids, more preferably composed of five or more amino acids, and preferably composed of ten or less amino acids.

In the peptide-conjugated resin, the content ratio of the peptide moiety is preferably 0.05 mol% or more, more preferably 0.1 mol% or more, still more preferably 0.15 mol% or more, and particularly preferably 0.2 mol% or more, and is preferably 25 mol% or less, more preferably 20 mol% or less, still more preferably 15 mol% or less, and particularly preferably 10 mol% or less. When the content ratio of the peptide moiety is the lower limit or more, the adhesiveness between the cell scaffold and cells after seeding can be further enhanced, and the proliferation rate of cells can be further enhanced. When the content ratio of the peptide moiety is the upper limit or less, the production cost can be suppressed.

The content ratio (mol%) of the peptide moiety is the amount of substance of the peptide moiety with respect to the total of the amount of substance of structural units constituting the peptide-conjugated resin.

The content ratio of the peptide moiety can be measured by, for example, nuclear magnetic resonance (NMR).

### <<Linker moiety>>

The linker moiety is a structural moiety derived from a linker. The linker moiety is usually located between the polyvinyl alcohol derivative moiety or the poly(meth) acrylic acid ester moiety and the peptide moiety. The polyvinyl alcohol derivative moiety or the poly(meth)acrylic acid ester moiety and the peptide moiety are bonded to each other via the linker moiety. The linker moiety is formed by a linker (crosslinking agent). Only one kind of the linker may be used, or two or more kinds thereof may be used in combination.

The linker is preferably a compound having a functional group capable of bonding to the peptide, and more preferably a compound having a functional group capable of condensing with a carboxyl group of the peptide or a functional group capable of condensing with an amino group.

Examples of the functional group capable of condensing with a carboxyl group of the peptide or the functional group capable of condensing with an amino group include a carboxyl group, a thiol group, an amino group, a hydroxyl group, and a cyano group.

From the viewpoint of favorably reacting with the peptide, the linker is preferably a compound having a carboxyl group or an amino group, and more preferably a compound having a carboxyl group.

In the case of obtaining a peptide-conjugated resin having a polyvinyl alcohol derivative moiety, examples of the linker having a carboxyl group include (meth)acrylic acid and carboxyl group-containing acrylamide. By using a carboxylic acid (carboxylic acid monomer) having a polymerizable unsaturated group as the linker having a carboxyl group, the carboxylic acid monomer can be polymerized by graft polymerization at the time of introduction of the linker, so that the number of carboxyl groups that can react with the peptide can be increased.

From the viewpoint of bonding the polyvinyl alcohol derivative and the peptide well, the linker is preferably (meth)acrylic acid, and more preferably acrylic acid.

In the case of obtaining a peptide-conjugated resin having a poly(meth)acrylic acid ester moiety, the linker preferably has a functional group capable of bonding to a (meth)acrylic acid ester. Examples of the functional group capable of bonding to a (meth)acrylic acid ester include a vinyl group, a (meth)acryloyl group, and an allyl group. The linker more preferably has a (meth)acryloyl group as the functional group capable of bonding to a (meth)acrylic acid ester, and is preferably a compound having a carboxyl group or an amino group and having a (meth)acryloyl group.

Examples of the linker in the case of obtaining a peptide-conjugated resin having a poly(meth)acrylic acid ester moiety include (meth)acrylic acid, itaconic acid, and acrylamide.

From the viewpoint of bonding the poly(meth)acrylic acid ester and the peptide well, the linker is preferably (meth)acrylic acid or itaconic acid, and more preferably (meth)acrylic acid.

### <Other details of cell scaffold>

The number average molecular weight of the peptide-conjugated resin is preferably 10000 or more, more preferably 50000 or more, and still more preferably 100000 or more, and is preferably 5000000 or less, more preferably 2500000 or less, and still more preferably 1000000 or less. When the number average molecular weight is the lower limit or more, the cell scaffold is less likely to be eluted into the liquid medium during cell culture, and the cell scaffold is less likely to be detached from the substrate or the like during cell culture. When the number average molecular weight is the upper limit or less, the solubility in an alcohol solvent can be enhanced.

The number average molecular weight of the peptide-conjugated resin can be measured by, for example, the following method. The peptide-conjugated resin is dissolved in tetrahydrofuran (THF) to prepare a 0.2 wt% solution of the peptide-conjugated resin. Next, evaluation is performed under the following measurement conditions using a gel permeation chromatography (GPC) measuring device (APC system, manufactured by Waters Corporation).
Column: HSPgel HR MB-M 6.0 × 150 mm
Flow rate: 0.5 mL/min
Column temperature: 40°C
Injection volume: 10 µL
Detector: RI, PDA
Standard sample: Polystyrene

The cell scaffold may contain a synthetic resin having no peptide moiety (synthetic resin to which no peptide is bonded) and a peptide-conjugated resin. The cell scaffold may contain a synthetic resin having the polyvinyl alcohol derivative moiety or the poly(meth) acrylic acid ester moiety and having no peptide moiety, and a peptide-conjugated resin. The cell scaffold may contain other components such as a resin having neither the polyvinyl alcohol derivative moiety nor the poly(meth)acrylic acid ester moiety. Examples of the other components include polyolefin resins, polyether resins, polyesters, epoxy resins, polyamide resins, polyimide resins, polyurethane resins, polycarbonate resins, celluloses, and polypeptides. Only one kind of the other components may be used, or two or more kinds thereof may be used in combination.

It is preferable that the cell scaffold contains the peptide-conjugated resin and does not contain or contains a synthetic resin to which no peptide is bonded.

The cell scaffold preferably has a layer containing the peptide-conjugated resin. The cell scaffold may have only a layer containing the peptide-conjugated resin, and may have a layer containing the peptide-conjugated resin and a layer containing a synthetic resin to which no peptide is bonded. From the viewpoint of more effectively exhibiting the effects of the present invention and the viewpoint of further enhancing the cell proliferation, a first surface of the layer containing the peptide-conjugated resin is preferably an upper surface of the cell scaffold. The peptide-conjugated resin is preferably present at least on a surface that adheres to somatic cells (adhesion surface with somatic cells).

The content of the peptide-conjugated resin in 100 wt% of the cell scaffold is preferably 90 wt% or more, more preferably 95 wt% or more, still more preferably 97.5 wt% or more, particularly preferably 99 wt% or more, and most preferably 100 wt% (total amount). When the content of the peptide-conjugated resin is the lower limit or more, the effects of the present invention can be more effectively exhibited. The content of the peptide-conjugated resin in 100 wt% of the cell scaffold may be 100 wt% or less or less than 100 wt%.

The total of the content of the peptide-conjugated resin and the content of the synthetic resin to which no peptide is bonded in 100 wt% of the cell scaffold is preferably 90 wt% or more, more preferably 95 wt% or more, still more preferably 97.5 wt% or more, particularly preferably 99 wt% or more, and most preferably 100 wt% (total amount). When the total content is the lower limit or more, the effects of the present invention can be more effectively exhibited. The total of the content of the peptide-conjugated resin and the content of the synthetic resin to which no peptide is bonded in 100 wt% of the cell scaffold may be 100 wt% or less or less than 100 wt%.

The cell scaffold preferably has a layer containing the cell adhesive protein. The cell scaffold may have only a layer containing the cell adhesive protein, and may have a layer containing the cell adhesive protein and a layer containing no cell adhesive protein. From the viewpoint of more effectively exhibiting the effects of the present invention and the viewpoint of further enhancing the cell proliferation, a first surface of the layer containing the cell adhesive protein is preferably an upper surface of the cell scaffold. The cell adhesive protein is preferably present at least on a surface that adheres to somatic cells (adhesion surface with somatic cells).

The content of the cell adhesive protein in 100 wt% of the cell scaffold is preferably 90 wt% or more, more preferably 95 wt% or more, still more preferably 97.5 wt% or more, particularly preferably 99 wt% or more, and most preferably 100 wt% (total amount). When the content of the cell adhesive protein is the lower limit or more, the effects of the present invention can be more effectively exhibited. The content of the cell adhesive protein in 100 wt% of the cell scaffold may be 100 wt% or less or less than 100 wt%.

The cell culture substrate including a cell scaffold containing a peptide-conjugated resin can be prepared, for example, by the following method (A) or (B). The following (A) is an example of a method for preparing a cell culture substrate by using a method of synthesizing a peptide-conjugated resin by a liquid phase method, and the following (B) is an example of a method for preparing a cell culture substrate by using a method of synthesizing a peptide-conjugated resin by a solid phase method.
(A) A solution containing a synthetic resin (hereinafter, referred to as "synthetic resin X") having a functional group capable of reacting with an amino group or a functional group capable of reacting with a carboxyl group, and a peptide is prepared. The synthetic resin X is reacted with the peptide to obtain a peptide-conjugated resin. A solution containing the peptide-conjugated resin and a solvent is applied onto a surface of a substrate, and then the solvent is volatilized.
(B) A solution containing a synthetic resin (synthetic resin X) having a functional group capable of reacting with an amino group or a functional group capable of reacting with a carboxyl group is prepared. The solution containing the synthetic resin X is applied onto a surface of a substrate. A liquid containing a peptide is added onto the surface of the substrate, and the synthetic resin X is reacted with the peptide.

Examples of the synthetic resin X include a (meth)acrylate polymer and a polyvinyl acetal derivative to which the linker is bonded.

The cell culture substrate including a cell scaffold containing a cell adhesive protein can be prepared, for example, by applying a solution containing a cell adhesive protein onto a surface of a substrate and then volatilizing a solvent.

Hereinafter, the details of the production method for iPS cells according to the present invention will be further described.

The production method for iPS cells includes, in the stated order: a seeding step of seeding somatic cells on a cell culture substrate including a cell scaffold; a removal step of removing suspended somatic cells without adhering to the cell scaffold; and an introducing step of introducing a reprogramming factor into somatic cells adhering to the cell scaffold. In the production method for iPS cells, the steps from the seeding step to the introducing step can be performed using the same cell culture substrate.

The production method for iPS cells preferably includes a culturing step (A) of culturing the seeded somatic cells between the seeding step and the removal step. The production method for iPS cells preferably includes the seeding step, the culturing step (A), the removal step, and the introducing step in this order.

The production method for iPS cells preferably includes a culturing step (B) of culturing the somatic cells into which the reprogramming factor has been introduced on the surface of the cell scaffold. The culturing step (B) is a step of culturing the somatic cells by using the cell culture substrate used in the introducing step. The production method for iPS cells preferably includes the seeding step, the removal step, the introducing step, and the culturing step (B) in this order. The production method for iPS cells more preferably includes the seeding step, the culturing step (A), the removal step, the introducing step, and the culturing step (B) in this order. In the production method for iPS cells, the steps from the seeding step to the culturing step (B) can be performed using the same cell culture substrate.

### (Seeding step)

The production method for iPS cells includes a step (seeding step) of seeding somatic cells on a cell culture substrate including a cell scaffold.

When the cell culture substrate is a cell culture container, the seeding density of the somatic cells in the seeding step is preferably 2×10³ cells or more, more preferably 5×10³ cells or more, and still more preferably 1×10⁴ cells or more, and is preferably 1×10⁸ cells or less, more preferably 1×10⁷ cells or less, and still more preferably 1×10⁶ cells or less, with respect to an area of 1 cm² of the upper surface of the cell scaffold.

When the cell culture substrate is a cell culture microcarrier or a cell culture fiber, the seeding density of the somatic cells in the seeding step is preferably 1×10³ cells or more, more preferably 2×10³ cells or more, and still more preferably 5×10³ cells or more, and is preferably 1×10⁷ cells or less, more preferably 1×10⁶ cells or less, and still more preferably 1×10⁵ cells or less, with respect to an area of 1 cm² of the outer surface of the cell scaffold.

### (Culturing step (A))

The production method for iPS cells preferably includes a step (culturing step (A)) of culturing the seeded somatic cells. In the culturing step (A), the somatic cells seeded in the seeding step are cultured. The culturing step (A) is preferably a step of pre-culturing the somatic cells.

As a medium for culturing the somatic cells in the culturing step (A), a conventionally known medium can be used. The medium is preferably a liquid medium. As the medium, a commercially available medium may be used, or a self-prepared medium may be used. As the medium, for example, the medium described in Non-Patent Document 1 described above can be used.

Examples of the commercially available products of the medium include "Stem Fit" manufactured by Ajinomoto Co., Inc., "StemSpan-ACF", "TeSR E8", and "mTeSR1" manufactured by STEMCELL Technologies Inc., "X-VIVO 10" manufactured by Lonza Corporation, and "StemFlex" and "Essential 8" manufactured by Thermo Fisher Scientific. As the medium, a medium obtained by adding various cytokines to the commercially available product may be used.

In the case of using peripheral blood mononuclear cells (PMBC) as the somatic cells, a medium containing IL-6 at 45 to 55 ng/mL, SCF at 45 to 55 ng/mL, TPO at 9 to 11 ng/mL, Flt-3L at 18 to 22 ng/mL, IL-3 at 18 to 22 ng/mL, and G-CSF at 9 to 11 ng/mL as a final concentration of cytokines is suitably used. The medium having the cytokine concentration can be obtained, for example, by adding these cytokines to the commercially available product so as to have the final concentration.

The culture temperature in the culturing step (A) is preferably 35°C or higher and preferably 38°C or lower. The CO₂ concentration during culture in the culturing step (A) is preferably 4% or more and preferably 6% or less. For example, the culture conditions in the culturing step (A) are conditions of 37°C and a CO₂ concentration of 5%.

The culture time of the culturing step (A) is preferably 3 days or more, more preferably 4 days or more, and still more preferably 5 days or more, and is preferably 8 days or less, more preferably 7 days or less, and still more preferably 6 days or less. The culture time in the culturing step (A) is the number of days after somatic cells are seeded in the seeding step. More specifically, the culture time in the culturing step (A) is the number of days from when somatic cells are seeded in the seeding step until suspended somatic cells without adhering to the cell scaffold are removed in the removal step. The day on which somatic cells are seeded in the seeding step is designated as Day 0, and the day after seeding is designated as Day 1.

During the culture in the culturing step (A), the medium may or may not be replaced. When the medium is replaced, it is preferable to perform replacement after somatic cells having adhesiveness among the somatic cells seeded in the seeding step are attached to the cell scaffold. Specifically, for example, when the medium is replaced during the culture in the culturing step (A), it is preferable to replace the medium on or after Day 3 from the start of the culture in the culturing step (A).

When the medium is replaced in the culturing step (A), it is preferable to replace 20 vol% or more of the medium, and it is more preferable to replace 40 vol% or more of the medium with respect to 100 vol% of the medium before the replacement per replacement of the medium.

### (Removal step)

The production method for iPS cells includes a step (removal step) of removing suspended somatic cells without adhering to the cell scaffold. Before the removal step, there are suspended somatic cells without adhering to the cell scaffold and somatic cells adhering to the cell scaffold. For example, when the culturing step (A) is performed, there are suspended somatic cells without adhering to the cell scaffold and somatic cells adhering to the cell scaffold. In the removal step, suspended somatic cells without adhering to the cell scaffold are removed.

In the removal step, examples of the method for removing the suspended somatic cells include a method for removing the suspended somatic cells together with a medium. Examples of the method for removing the suspended somatic cells together with a medium include a method using a pipette. The suspended somatic cells may be washed with a buffer such as PBS and a liquid such as a medium before or after being removed together with the medium.

From the viewpoint of easily removing the suspended somatic cells, in the removal step, the method for removing the suspended somatic cells is preferably a method for removing the suspended somatic cells together with a medium. In the removal step, it is preferable to remove the suspended somatic cells by removing the medium containing the suspended somatic cells.

In the removal step, it is preferable to remove 50% or more of somatic cells, it is more preferable to remove 70% or more of somatic cells, it is further preferable to remove 90% or more of somatic cells, it is particularly preferable to remove 95% or more of somatic cells, and it is most preferable to remove 99% or more of somatic cells on the basis of the number of cells among the suspended somatic cells. In this case, the effects of the present invention can be more effectively exhibited. In the removal step, 100% or less of somatic cells may be removed, less than 100% of somatic cells may be removed, or 99% or less of somatic cells may be removed on the basis of the number of cells among the suspended somatic cells.

When the medium is removed in the removal step, it is preferable to remove 10 vol% or more of the medium, it is more preferable to remove 20 vol% or more of the medium, it is still more preferable to remove 40 vol% or more of the medium, it is further preferable to remove 60 vol% or more of the medium, it is particularly preferable to remove 80 vol% or more of the medium, and it is most preferable to remove 90 vol% or more of the medium with respect to 100 vol% of the medium before removing the medium. When the ratio of the medium to be removed is the lower limit or more, more suspended somatic cells can be removed, and thus the effects of the present invention can be more effectively exhibited. When the medium is removed in the removal step, 100 vol% or less of the medium may be removed, less than 100 vol% of the medium may be removed, 95 vol% or less of the medium may be removed, or 90 vol% or less of the medium may be removed with respect to 100 vol% of the medium before removal of the medium.

The time from when somatic cells are seeded in the seeding step to when the suspended somatic cells are removed in the removal step is preferably 1 day or more, more preferably 2 days or more, still more preferably 3 days or more, further preferably 4 days or more, and particularly preferably 5 days or more, and is preferably 15 days or less, more preferably 10 days or less, still more preferably 8 days or less, further preferably 7 days or less, and particularly preferably 6 days or less. When the time is the lower limit or more and the upper limit or less, the effect of the present invention can be more effectively exhibited. The time is the number of days after somatic cells are seeded in the seeding step. The day on which somatic cells are seeded in the seeding step is designated as Day 0, and the day after seeding is designated as Day 1.

### (Introducing step)

The production method for iPS cells includes a step (introducing step) of introducing a reprogramming factor into somatic cells adhering to the cell scaffold. In the introducing step, a reprogramming factor is introduced into somatic cells (somatic cell population) adhering to the cell scaffold after the removal step. That is, in the introducing step, a reprogramming factor is introduced into somatic cells (somatic cell population) remaining on the surface of the cell scaffold after the removal step.

In the introducing step, a reprogramming factor can be introduced into somatic cells adhering to the cell scaffold without collecting the somatic cells adhering to the cell scaffold. Therefore, iPS cells can be easily produced by a simple process.

In the introducing step, somatic cells adhering to the cell scaffold preferably include cells having phagocytosis, more preferably include monocytes, macrophages, or dendritic cells, and still more preferably include monocytes or macrophages. In this case, iPS cells having favorable quality can be obtained in a short period of time.

As the reprogramming factor (nuclear reprogramming factor), a conventionally known reprogramming factor can be used.

Examples of the gene included in the reprogramming factor include Oct3/4, Sox2, Sox1, Sox3, Sox15, Sox17, Klf4, Klf2, c-Myc, N-Myc, L-Myc, Nanog, Lin28, Fbx15, ERas, ECAT15-2, Tcl1, beta-catenin, Lin28b, Sall1, Sall4, Esrrb, Nr5a2, Tbx3, and Glis1. Only one kind of the reprogramming factor may be used, or two or more kinds thereof may be used in combination.

The reprogramming factor preferably includes an Oct family gene, a Sox family gene, a Klf family gene, a Myc family gene, a Lin28 family gene, or a Nanog family gene.

The reprogramming factor more preferably includes an Oct family gene, a Klf family gene, and a Sox family gene, still more preferably includes Oct3/4, Sox2, and Klf4, further preferably includes Oct3/4, Sox2, Klf4, and c-Myc, and further preferably includes Oct3/4, Sox2, Klf4, c-Myc, Lin28, and Nanog.

As a method for introducing the reprogramming factor into somatic cells, a conventionally known introduction method can be used.

Examples of the method for introducing the reprogramming factor into somatic cells include a method using a vector and a method using mRNA. Examples of the vector include viral vectors such as a sendaiviral vector, a retroviral vector, a lentiviral vector, an adenoviral vector, and an adeno-associated viral vector, and non-viral vectors such as an episomal vector and a plasmid vector.

In the introducing step, it is more preferable to use a vector, it is more preferable to use a viral vector, and it is still more preferable to use a sendaiviral vector. That is, in the introducing step, it is still more preferred a reprogramming factor is introduced into somatic cells adhering to the cell scaffold using a sendaiviral vector. In this case, safety can be enhanced.

Examples of the commercially available products of the sendaiviral vector include "CytoTune-iPS" series manufactured by ID Pharma, and "SRV iPSC Vector" series manufactured by TOKIWA-Bio inc.

In the production method for iPS cells, in the introducing step, as compared with the conventional method, iPS cells can be established with a smaller amount of viral vector. In the introducing step, the viral vector (particularly, the sendaiviral vector) is infected with preferably Multiplicity of Infection (MOI) of 3 or less, more preferably an MOI of 2 or less, and further preferably an MOI of 1 or less, and is infected with preferably an MOI of 0.001 or more, and more preferably an MOI of 0.01 or more. In the introducing step, the viral vector (particularly, the sendaiviral vector) is infected with preferably an MOI of 0.001 or more and 3 or less, more preferably an MOI of 0.2 or more and 3 or less, and further preferably an MOI of 0.5 or more and 2 or less. The infection time is preferably 15 minutes or more and 4 hours or less, and more preferably 30 minutes or more and 4 hours or less. The MOI in the present specification means the amount of the viral vector relative to the number of somatic cells seeded in the seeding step.

In the introducing step, as the reprogramming factor, mRNA is also preferably used. The reprogramming factor is also preferably mRNA. That is, in the introducing step, a reprogramming factor may be introduced into somatic cells adhering to the cell scaffold using mRNA. In this case, iPS cells can be produced even with a smaller amount of mRNA. In the case of using mRNA, as compared with the case of using a viral vector as the reprogramming factor, safety can be enhanced.

In the introducing step, in the case of using mRNA as the reprogramming factor, examples of a method for introducing a reprogramming factor (mRNA) into somatic cells include a plasma gene introduction method, an electroporation method, and a lipofection method.

The introducing step is performed preferably within 2 minutes, more preferably within 1 minute, and still more preferably within 0.5 minutes after the suspended somatic cells are removed in the removal step. In this case, iPS cells having favorable quality can be obtained in a shorter period of time. The phrase "the introducing step is performed within X minutes after the suspended somatic cells are removed in the removal step" means that the time from when the suspended somatic cells are removed in the removal step to when the somatic cells adhering to the cell scaffold come into contact with a component (vector or the like) containing a reprogramming factor is within X minutes.

### (Culturing step (B))

The production method for iPS cells preferably includes a step (culturing step (B)) of culturing the somatic cells (somatic cell population) into which the reprogramming factor has been introduced on the surface of the cell scaffold. In the culturing step (B), the somatic cells (somatic cell population) obtained in the introducing step are cultured. In the culturing step (B), adhesion culture is preferably performed.

As a medium for culturing the somatic cells in the culturing step (B), a conventionally known medium can be used. The medium is preferably a liquid medium. As the medium, a commercially available medium may be used, or a self-prepared medium may be used. As the medium, for example, the medium described in Non-Patent Document 1 described above can be used.

Examples of the commercially available products of the medium include "Stem Fit" manufactured by Ajinomoto Co., Inc., "StemSpan-ACF", "TeSR-E8", and "mTeSR1" manufactured by STEMCELL Technologies Inc., "X-VIVO 10" manufactured by Lonza Corporation, and "StemFlex" and "Essential 8" manufactured by Thermo Fisher Scientific. As the medium, a medium obtained by adding various cytokines of the commercially available product may be used.

The culture temperature in the culturing step (B) is preferably 36°C or higher and preferably 38°C or lower. The CO₂ concentration during culture in the culturing step (B) is preferably 4% or more and preferably 6% or less. For example, the culture conditions in the culturing step (B) are conditions of 37°C and a CO₂ concentration of 5%. During the culture in the culturing step (B), the medium may or may not be replaced. When the medium is replaced, for example, the medium can be replaced with a fresh medium at a frequency of once every two days.

In the culturing step (B), iPS cells may or may not be established. Whether or not iPS cells are established may not necessarily be confirmed at the end of the culturing step (B). In the production method for iPS cells according to the present invention, iPS cells are induced in, for example, about 14 days from the start of culture in the culturing step (B), and a cell population including iPS cells can be obtained.

### (Subculturing step)

From the viewpoint of obtaining a cell population including iPS cells having more favorable quality, the production method for iPS cells preferably includes a subculturing step of subculturing the cells (cell population) obtained in the culturing step (B).

In the subculturing step, the same type of cell culture substrate as the cell culture substrate used from the seeding step to the culturing step (B) may be used, or a different type of cell culture substrate may be used.

Examples of a passaging method in the subculturing step include a method of performing colony picking and a method using a cell detachment agent.

From the viewpoint of easy passaging, the passaging method in the subculturing step is preferably a method using a cell detachment agent. In the conventional production method for iPS cells, it is necessary to perform colony picking particularly at the time of the first passage, but in the production method for iPS cells of the present invention, since the removal step and the introducing step are provided, the cells can be passaged using the cell detachment agent even at the time of the first passage.

Examples of the commercially available products of the cell detachment agent include "TrypLE Select" and "TrypLE Express" manufactured by Thermo Fisher Scientific.

At the time of cell culture in the subculturing step, adhesion culture is preferably performed.

As the medium usable in the subculturing step, a conventionally known medium can be used. The medium is preferably a liquid medium. As the medium, a commercially available medium may be used, or a self-prepared medium may be used. As the medium, for example, the medium described in Non-Patent Document 1 described above can be used. As the medium, the medium described in the section of the culturing step (B) can be used.

The culture temperature of the subculturing step is preferably 36°C or higher and preferably 38°C or lower. The CO₂ concentration during culture in the subculturing step is preferably 4% or more and preferably 6% or less. For example, the culture conditions in the subculturing step are conditions of 37°C and a CO₂ concentration of 5%. During the culture in the subculturing step, the medium may or may not be replaced. When the medium is replaced, for example, the medium can be replaced with a fresh medium at a frequency of once every two days.

The number of passages of the cells in the subculturing step may be 1, 2, 2 or more, 3, 3 or more, 4 or more, 5 or more, 6 or more, or 7 or more, and may be 10 or less, 9 or less, 8 or less, 7 or less, 6 or less, 5 or less, or 4 or less.

In the conventional production method for iPS cells, it is necessary to repeat cell passaging in order to obtain iPS cells having favorable quality, but in the production method for iPS cells according to the present invention, iPS cells having favorable quality can be obtained even when the number of passages of the cells is small. Therefore, in the production method for iPS cells according to the present invention, iPS cells having favorable quality can be obtained in a short period of time.

Whether or not somatic cells have been induced into iPS cells can be confirmed, for example, by observing the morphology of the cells or analyzing whether or not an undifferentiated marker is expressed in the cells by a flow cytometer. As the undifferentiated marker, TRA-1-60, SSEA4, and the like are known. In particular, since TRA-1-60 is an antigen specific to iPS cells or ES cells and is not detected in somatic cells, it is a suitable marker for confirming whether or not it is induced in iPS cells (for example, WO 2018/155595 A1, JP 2019-162054 A, and Elayne M Chan1 et al. (Live cell imaging distinguishes bona fide human iPS cells from partially reprogrammed cells, nature biotechnology volume 27 number 11 November 2009)).

Hereinafter, the present invention will be specifically described with reference to Examples. The present invention is not limited to the following Examples.

As the cell culture substrate, the following cell culture containers and cell culture microcarriers were prepared.

Culture container (P) (a plate including a cell scaffold containing a peptide-conjugated polyvinyl acetal resin, prepared by the following "Preparation method for culture container (P)")

Microcarrier (P) (a microcarrier including a cell scaffold containing a peptide-conjugated polyvinyl acetal resin, prepared by the following "Preparation method for microcarrier (P)")

Culture container (L) (a plate coated with "Laminin 511E8 fragment (iMatrix-511)" manufactured by Nippi, Inc., prepared by the following "Preparation method for culture container (L)")

In the following tables, the "peptide-conjugated polyvinyl acetal resin" which is a cell scaffold provided in the culture container (P) and the microcarrier (P) was described as "PVB-AA", and "Laminin 511E8 fragment (iMatrix-511)" which is a cell scaffold provided in the culture container (L) was described as "Laminin".

### [Preparation method for culture container (P)]

### Preparation of coating solution containing peptide-conjugated polyvinyl acetal resin:

As a polyvinyl acetal resin, a polyvinyl butyral resin having an acetalization degree (butyralization degree) of 65 mol%, a hydroxyl group amount of 32 mol%, and an acetyl group amount of 3 mol% was prepared. Acrylic acid (30 parts by weight) and a polyvinyl acetal resin (70 parts by weight) were dissolved in 255 parts by weight of tetrahydrofuran to obtain a polymer mixed solution. PERBUTYL O (0.015 parts by weight) (manufactured by NOF CORPORATION) was dissolved in the obtained polymer mixed solution, and the mixture was reacted at 90°C for 6 hours. Next, the solution after the reaction was mixed with 30000 parts by weight of water. The obtained precipitate was vacuum-dried at 80°C for 3 hours to prepare a polyvinyl acetal resin having a linker (acrylic acid) (polyvinyl butyral resin having a linker). Hereinafter, the "polyvinyl acetal resin having a linker" may be referred to as the "polyvinyl acetal resin (X)".

Dimethylformamide (DMF) was prepared as a first solvent. Dimethylformamide (DMF) was prepared as a second solvent. As the peptide, a cyclic peptide having an amino acid sequence of Arg-Gly-Asp-Phe-Lys (five amino acid residues, forming a cyclic skeleton by bonding Arg and Lys, Phe is a D form) was prepared. As a condensing agent, 1-ethyl-3-(3-dimethylaminopropyl)carbodiimide hydrochloride was prepared. The polyvinyl acetal resin (X) (50 parts by weight) and the peptide (2 parts by weight) were mixed with 1000 parts by weight of the first solvent to prepare a first solution. A condensing agent (1 part by weight) was mixed with 1000 parts by weight of the second solvent to prepare a second solution. The first solution and the second solution were mixed to prepare a solution containing the polyvinyl acetal resin (X), the peptide, and the condensing agent.

The obtained solution was reacted at 40°C for 2 hours, and a carboxyl group in the structural unit derived from acrylic acid of the polyvinyl acetal resin (X) and an amino group of Lys of the peptide were dehydrated and condensed, thereby obtaining a solution containing a peptide-conjugated polyvinyl acetal resin.

The obtained solution containing a peptide-conjugated polyvinyl acetal resin was diluted 100 times with DMF, and added dropwise to a column packed with an ion exchange resin (manufactured by Organo Corporation) at a rate of 0.3 mL/min for washing. The solution after washing was vacuum-dried at 60°C for 3 hours to obtain a dried solid, the dried solid was dissolved in butanol (alcohol solvent), and then 5 parts by weight of acetic acid (pH adjusting agent) was added to 100 parts by weight of butanol (alcohol solvent) to obtain a coating solution containing a peptide-conjugated polyvinyl acetal resin and butanol. The content of the peptide-conjugated polyvinyl acetal resin in the coating solution was set to 0.1 wt%.

### Preparation of culture container (P):

To each well of a 96-well plate (bottom area of each well: 0.32 cm²), 20 µL of the obtained coating solution was applied by a cast coating method, and then the alcohol solvent was removed by vacuum drying at 60°C for 3 hours. In this way, a culture container (P) in which a cell scaffold containing a peptide-conjugated polyvinyl acetal resin (a resin film which is a dried product layer of the coating solution) was disposed on the bottom surface of each well was obtained. In the peptide-conjugated polyvinyl acetal resin, the content ratio of the peptide moiety was 1 mol%. The number average molecular weight of the peptide-conjugated polyvinyl acetal resin was 50000.

### [Preparation method for microcarrier (P)]

As the base particle, divinylbenzene particles having an average particle size of 300 um and a particle size CV value of 1% were prepared. The polyvinyl acetal resin (X) (1 part by weight) was dissolved in butanol (19 parts by weight). The base particle (1 part by weight) was added to the obtained solution, and the mixture was stirred, then filtered, washed with pure water, and vacuum-dried at 60°C for 5 hours to obtain a polyvinyl acetal resin-coated particle having a linker moiety formed thereon. The peptide (a cyclic peptide having an amino acid sequence of Arg-Gly-Asp-Phe-Lys (five amino acid residues, forming a cyclic skeleton by bonding Arg and Lys, Phe is a D form)) was prepared. The peptide (1 part by weight) and 1-ethyl-3-(3-dimethylaminopropyl)carbodiimide hydrochloride (condensing agent) (1 part by weight) were added to phosphate buffered saline containing neither calcium nor magnesium so that the final concentration of the peptide was 1 mM, thereby preparing a peptide-conjugated solution. The polyvinyl acetal resin-coated particles (1 part by weight) having a linker moiety formed thereon were added to the obtained peptide-conjugated solution (20 parts by weight), so that a carboxyl group of the linker moiety and an amino group of Lys of the peptide were dehydration-condensed. The obtained suspension was filtered, washed with pure water, and vacuum-dried at 60°C for 5 hours. In this way, a microcarrier (P) in which a cell scaffold containing a peptide-conjugated polyvinyl acetal resin was disposed on the outer surface of the base particle was obtained. In the peptide-conjugated polyvinyl acetal resin, the content ratio of the peptide moiety was 1 mol%. The number average molecular weight of the peptide-conjugated polyvinyl acetal resin was 50000.

### [Preparation method for culture container (L)]

"Laminin 511E8 fragment (iMatrix-511, 0.5 mg/mL)" manufactured by Nippi, Inc. was diluted 300 times with phosphate buffered saline (PBS (Ca-/Mg-)), and the resulting solution was added to each well of a 96-well plate (bottom area of each well: 0.32 cm²) so as to be 0.5 µg/cm². The 96-well plate was left to stand still in an incubator at 37°C and a CO₂ concentration of 5% for 1 hour, and then the supernatant was removed to obtain a culture container (L) in which Laminin 511E8 fragment was disposed on the bottom surface of each well.

As the medium, the following were prepared.

Medium (1) (prepared by the following "Preparation method for medium (1)", corresponding to mononuclear culture medium for non-T cells described in Non-Patent Document 1)

Medium (2) (prepared by the following "Preparation method for medium (2)", corresponding to maintenance culture medium StemFit described in Non-Patent Document 1)

### [Preparation method for medium (1)]

"Liquid A for Stem Fit AK02N" (400 mL) manufactured by Ajinomoto Co., Inc. and "Liquid B for Stem Fit AK02N" (100 mL) manufactured by Ajinomoto Co., Inc. were mixed. To the obtained mixed solution, IL-6, SCF, TPO, Flt-3L, IL-3, and G-CSF were added at final concentrations of 50 ng/mL, 50 ng/mL, 10 ng/mL, 20 ng/mL, 20 ng/mL, and 10 ng/mL, respectively. In this way, a medium (1) was obtained.

### [Preparation method for medium (2)]

"Liquid A for Stem Fit AK02N" (400 mL) manufactured by Ajinomoto Co., Inc., "Liquid B for Stem Fit AK02N" (100 mL) manufactured by Ajinomoto Co., Inc., and "Liquid C for Stem Fit AK02N" (2 mL) manufactured by Ajinomoto Co., Inc. were mixed. In this way, a medium (2) was obtained.

In the following Examples, cell culturing and introduction of a reprogramming factor were performed according to the protocol described in Non-Patent Document 1 except for the parts particularly described below.

### (Example 1)

### <Seeding step>

As the somatic cells, peripheral blood mononuclear cells ("Normal Human PBMC-Japanese Donor Purified" manufactured by Precision for Medicine) were prepared. The medium (1) (150 µL) per well was added to the culture container (P). Next, peripheral blood mononuclear cells were seeded on the culture container (P) at a seeding amount of 8.0×10⁴ cells/well.

### <Culturing step (A)>

The cells seeded on the culture container (P) were cultured in an incubator at 37°C and a CO₂ concentration of 5% for 5 days. On Day 3 from the start of culture, 75 µL of the liquid medium in the well was recovered from a position close to the liquid level using a pipette so as not to remove suspended somatic cells as much as possible, and then replaced with 75 µL of a fresh liquid medium.

### <Removal step>

On Day 5 from the start of culture (Day 5 after somatic cells were seeded in the seeding step), all the suspended somatic cells without adhering to the cell scaffold were removed together with the liquid medium in the culture container.

### <Introducing step>

A sendaiviral vector ("SRV^{™}iPSC-4 Vector" manufactured by TOKIWA-Bio inc.) and the medium (1) were mixed so that MOI was 0.75 and the total amount was 50 µL, thereby obtaining a viral vector-containing solution. The viral vector-containing solution was added to each well of the culture container (P) after the removal step so that the entire inner surface of the well was immersed. The addition of the viral vector-containing solution to each well of the culture container (P) was performed within 0.5 minutes after the suspended somatic cells were removed in the removal step. The culture container (P) to which the viral vector-containing solution was added was left to stand still in an incubator at 37°C and a CO₂ concentration of 5% for 2 hours. Then, 150 µL of fresh medium (1) was added to each well, and a washing operation of removing 150 µL of the medium was performed 3 times in total. After the washing operation, 100 µL of fresh medium (1) was added to each well. In this way, a reprogramming factor was introduced into somatic cells.

### <Culturing step (B)>

The fresh medium (2) (70 µL) was added to each well of the culture container (P), and somatic cells into which the reprogramming factor had been introduced were subjected to adhesion culture in an incubator at 37°C and a CO₂ concentration of 5%. On Day 3 from the start of the culture in the culturing step (B), 70 µL of fresh medium (2) was added to each well of the culture container (P). On Day 5 and Day 7 from the start of the culture in the culturing step (B), 70 µL of the medium was removed from each well of the culture container (P), and 70 µL of fresh medium (2) was added. After Day 9 from the start of culture in the culturing step (B), the liquid in the well was replaced with 70 µL of fresh medium (2) at a frequency of once or twice every two days.

### <Subculturing step>

The cell population on Day 14 from the start of the culturing step (B) was defined as single cells using a cell detachment agent ("TrypLE Select" manufactured by Thermo Fisher Scientific). From the obtained single cells, the total number of cells present in each well of the cell culture container was calculated by a fully automated cell counter. Cells were passaged by seeding cells of 1×10⁴ cells to 50×10⁴ cells in a new culture container (P) on the basis of the calculated total cell number. The cells were passaged once or twice a week, and the medium (2) was used as the medium. At the time of passage, a ROCK inhibitor (Y-27632) was added to the medium (2) so as to have a final concentration of 10 µmol/L. After the day after the passage, the medium was replaced at a frequency of once or more every three days.

### (Example 2)

The steps from the seeding step to the subculturing step were performed in the same manner as in Example 1 except that the culture container (L) was used instead of the culture container (P).

### (Example 3)

The steps from the seeding step to the subculturing step were performed in the same manner as in Example 1 except that monocytes ("human peripheral blood-derived CD14-positive monocytes" manufactured by KAC Co., Ltd.) were used as somatic cells and the seeding amount in the seeding step was changed as described in the following table.

### (Example 4)

As a pretreatment, the microcarrier (P) was immersed in PBS for 30 minutes. The microcarrier (P) subjected to the pretreatment was added to each well of a 96-well plate (bottom area of each well: 0.32 cm²) so that there were two to three layers of the microcarrier (P) in the well. The steps from the seeding step to the culturing step (B) were performed in the same manner as in Example 1 except that the seeding amount in the seeding step was changed as described in the following table and the adhesion culture was performed on the surface of the microcarrier (P) using this 96-well plate.

The subculturing step was performed in the same manner as in Example 1 except for the following.

On Day 21 from the start of the culturing step (B), 100 µL of a cell detachment agent ("TrypLE Express" manufactured by Thermo Fisher Scientific) was added to each well, and the plate was left to stand still for 10 minutes. Next, pipetting was performed 20 to 30 times, the microcarrier (P) was separated with a cell filter (manufactured by Corning Incorporated, 40 µm pore size) set in a tube, and only cells were recovered in the tube. After a new microcarrier (P) was prepared and subjected to a pretreatment in the same manner as described above, the microcarrier (P) subjected to the pretreatment was added to each well of a 48-well plate so that there were two to three layers of the microcarrier (P) in the well. The recovered cells were seeded in a 48-well plate at 1.25×10⁴ cells/well to passage the cells.

### (Comparative Example 1)

In Comparative Example 1, somatic cells adhering to the cell scaffold were recovered, and a reprogramming factor was introduced into the recovered somatic cells. Specifically, iPS cells were established by the following procedure.

### <Seeding step and culturing step (A)>

The steps from the seeding step to the culturing step (A) were performed in the same manner as in Example 1 except that the seeding amount in the seeding step was changed as described in the following table.

### <Recovery step>

After removing all the culture supernatant, each well was washed twice with 1 mL of PBS. Then, somatic cells adhering to the cell scaffold were detached with a cell detachment agent ("TrypLE Select" manufactured by Thermo Fisher Scientific) and recovered in a 15 mL tube.

### <Introducing step>

The somatic cells recovered in the 15 mL tube were housed in a 1.5 mL tube with the number of cells of 3×10⁵ cells, and the 1.5 mL tube was centrifuged under the conditions of 300×g for 5 minutes. After centrifugation, the supernatant was removed and an appropriate amount of the medium (1) was added to the 1.5 mL tube. Then, a sendaiviral vector ("SRV^{™}iPSC-4 Vector" manufactured by TOKIWA-Bio inc.) was added to the 1.5 mL tube so that MOI was 3. Then, the 1.5 mL tube was left to stand still in an incubator at 37°C for 2 hours. Next, 1 mL of the medium (1) was added to the 1.5 mL tube, and then centrifuged under the conditions of 300×g for 5 minutes. After centrifugation, the supernatant was removed and tapped. The steps of addition of the medium (1), centrifugation, and removal of the supernatant described above were repeated twice more. Next, 0.1 mL of the medium (1) was added to the 1.5 mL tube and mixed well. In this way, a reprogramming factor was introduced into somatic cells.

### <Culturing step (B)>

The medium (2) (0.75 mL) per well was added to the culture container (P). The somatic cells obtained in the introducing step into which the reprogramming factor had been introduced were seeded on the cell scaffold in the culture container (P) at the number of cells of 1.5×10⁵ cells/well, and subjected to adhesion culture in an incubator at 37°C and a CO₂ concentration of 5%. On Day 1, Day 3, Day 5, and Day 7 from the start of the culture in the culturing step (B), 0.5 mL of fresh medium (2) was added to the culture container (P). After Day 9 from the start of culture in the culturing step (B), the liquid in the culture container (P) was replaced with 0.75 mL of fresh medium (2) at a frequency of once or more every two days.

### <Subculturing step>

The cell population obtained in the culturing step (B) was subjected to the subculturing step in the same manner as in Example 1.

### (Comparative Example 2)

The steps from the seeding step to the subculturing step were performed in the same manner as in Comparative Example 1 except that suspended somatic cells without adhering to the cell scaffold were recovered after the culturing step (A) and the introducing step was performed using the recovered somatic cells.

In all of Examples 1 to 4 and Comparative Examples 1 and 2, when the colony morphology was confirmed on Day 14 from the start of culture in the culturing step (B), the colony morphology of iPS cells was confirmed.

### (Evaluation)

In the following description and drawings, the number used together with P means the number of passages from the start of the subculturing step. P0 is passage 0, and is a cell after the culturing step (B) (a cell before being subjected to the subculturing step).

### (1) Flow cytometry measurement of cells before viral infection

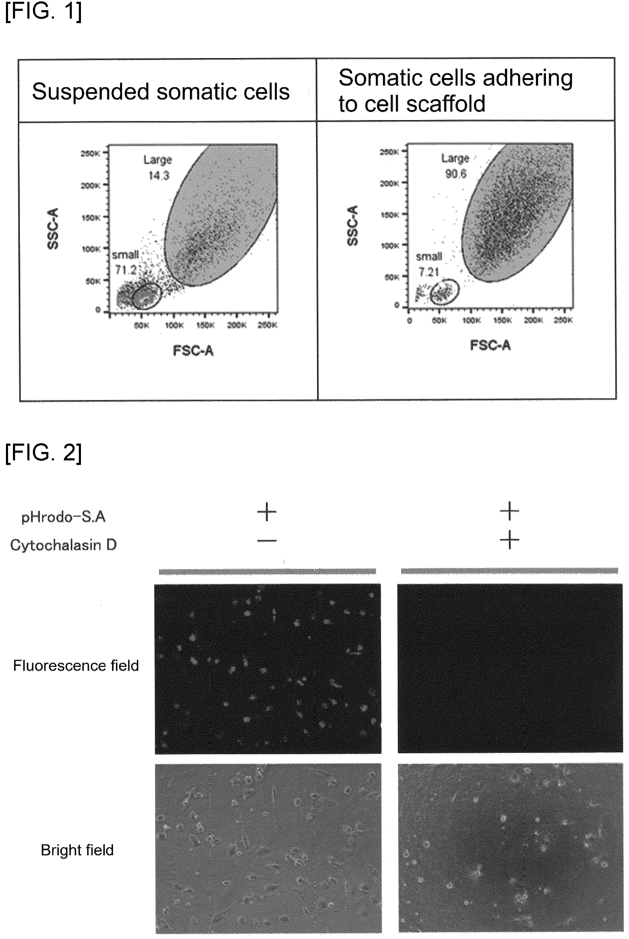

In Example 1, each of the somatic cells (suspended somatic cells) removed in the removal step and the somatic cells adhering to the cell scaffold after the removal step was measured by flow cytometry ("Attune NxT Flow Cytometer" manufactured by Thermo Fisher Scientific). The sample of somatic cells adhering to the cell scaffold for flow cytometry was obtained by washing the well twice with 1 mL of PBS and then peeling the well with a cell detachment agent ("TrypLE Select" manufactured by Thermo Fisher Scientific). Results of FSC-SSC dot plot are shown in Fig. 1. As shown in Fig. 1, as compared with the suspended somatic cells, somatic cells adhering to the cell scaffold had significantly more cells having a property in which both FSC and SSC are large.

### (2) Phagocytosis of somatic cells adhering to cell scaffold

In Example 1, the phagocytosis of somatic cells adhering to the cell scaffold after the removal step was evaluated by the following procedure. pHrodo Green S. aureus BioParticles conjugates (manufactured by Life Technologies) was added to the medium (1) so as to have a final concentration of 0.1 mg/mL, and 750 µL of pHrodo Green S. aureus BioParticles conjugates was added to the wells of the culture container after the removal step. Next, the mixture was left to stand still in an incubator at 37°C and a CO₂ concentration of 5% for 90 minutes. Next, using a fluorescence microscope ("BZ-X800" manufactured by KEYENCE CORPORATION), the wells were observed in each of the fluorescence field and the bright field at a magnification of 200 times. As a negative control, evaluation was also performed according to the following procedure. First, cytochalasin D was added to the medium (1) so as to have a final concentration of 10 µM, and 750 µL of cytochalasin D was added to the wells of the culture container after the removal step. Next, the mixture was left to stand still in an incubator at 37°C and a CO₂ concentration of 5% for 30 minutes. Next, pHrodo Green S.aureus BioParticles conjugates were added to the wells so as to have a final concentration of 0.1 mg/mL, and the mixture was left to stand still in an incubator at 37°C and a CO₂ concentration of 5% for 90 minutes. Next, the wells were observed under the same observation conditions as described above. Results are shown in Fig. 2. As shown in Fig. 2, in the fluorescence field, cells adhering to the cell scaffold were observed only in the absence of cytochalasin D. pHrodo Green S.aureus BioParticles conjugates do not exhibit fluorescence outside the phagosome, but exhibit fluorescence inside the phagosome. Cytochalasin D acts as an inhibitor of phagocytosis. Therefore, it was confirmed that the cells adhering to the cell scaffold after the removal step were cells having phagocytosis.

### (3) Morphological observation of cells after viral infection

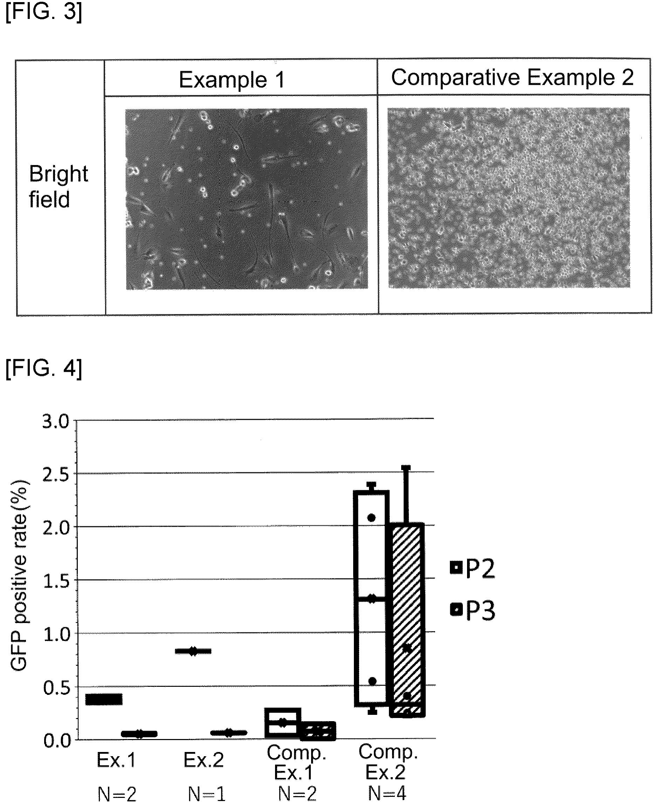

In Example 1 and Comparative Example 2, the morphology of cells on Day 1 (Day 1 after infection with virus) after the somatic cells into which the reprogramming factor had been introduced were cultured was observed according to the following procedure. The wells of the culture container on Day 1 from starting of the culturing step (B) were washed once with 1×DPBS. Using a fluorescence microscope ("BZ-X800" manufactured by KEYENCE CORPORATION), the wells were observed in the bright field at a magnification of 100 times, and captured. Results are shown in Fig. 3. As shown in Fig. 3, in Example 1, a large number of cells having a large size and an elongated shape were observed as compared with Comparative Example 2.

### (4) Residual rate of virus

In Examples 1 to 3 and Comparative Examples 1 and 2, the residual rate of virus was evaluated by the following procedure. Some of single cells were recovered in a 1.5 mL tubes at cell passaging. The recovered single cells were measured by flow cytometry ("Attune NxT Flow Cytometer" manufactured by Thermo Fisher Scientific). The percentage of GFP-positive cells included in the main cell population developed in FSC-SSC dot plot was analyzed. Results are shown in Tables 1 to 3 and Fig. 4. Fig. 4 shows results of Examples 1 and 2 and Comparative Examples 1 and 2 and the number N. In Fig. 4, data of P2 and P3 are shown in order from the left. The smaller the GFP positive rate on the vertical axis in Fig. 4, the smaller the residual rate of virus. As shown in Tables 1 to 3 and Fig. 4, in Examples 1 to 3, the result of the virus residual rate was particularly excellent, and the time until disappearance of the virus was short.

### (5) Undifferentiation property (TRA-1-60 positive rate)

An antibody against TRA-1-60, which was labeled with a PE fluorescent dye ("TRA-1-60 PE Antibody" manufactured by Thermo Fisher Scientific) was prepared. Each of the cells P2 and P4 was contacted with this antibody. Next, the positive rate of TRA-1-60 was determined using flow cytometry ("Attune NxT Flow Cytometer" manufactured by Thermo Fisher Scientific). Specifically, in Examples 1 to 3 and Comparative Examples 1 and 2, evaluation was carried out by the following procedure.

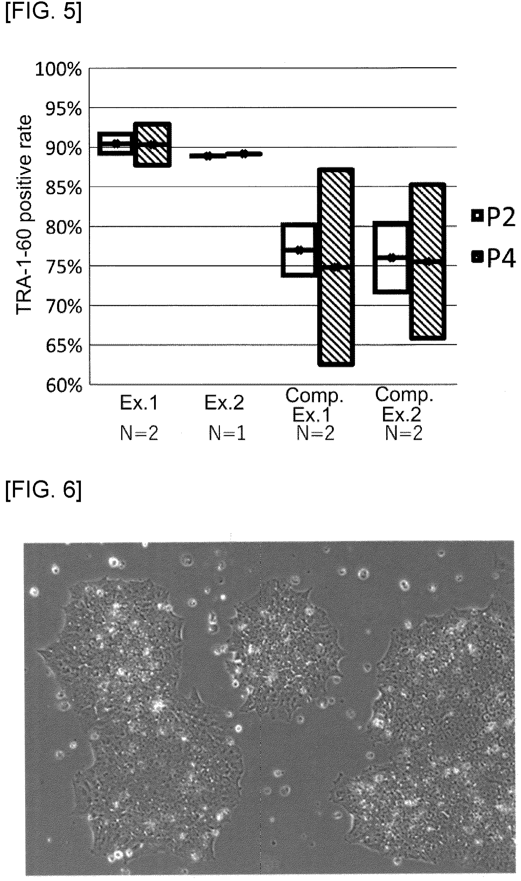

The cells of P2 and P4 were made into single cells using an detachment agent ("TrypLE Select" manufactured by Thermo Fisher Scientific). The obtained single cells were subjected to a fully automated cell counter, and the cell concentration was calculated. Then, cells of 5×10⁵ cells were collected in a 1.5 mL tube, and the 1.5 mL tube was centrifuged under the conditions of 300×g for 5 minutes. After centrifugation, the supernatant was removed and 1×DPBS was added to the 1.5 mL tube. Subsequently, centrifugation and removal of the supernatant were performed again under the above conditions. Blocking was then performed in 1 wt% BSA-containing DPBS. Next, an antibody solution containing the above-described antibody ("TRA-1-60 PE Antibody" manufactured by Thermo Fisher Scientific) in an appropriate amount was added to the 1.5 mL tube, and the mixture was incubated in a dark place and at room temperature for 1 hour. Next, the 1.5 mL tube was centrifuged under the conditions of 300×g for 5 minutes before the supernatant was removed and washed twice with 1×DPBS. Next, 0.4 mL of PBS containing 1 wt% BSA was added to the 1.5 mL tube, mixed, and measured by flow cytometry. The TRA-1-60-positive cells (TRA-1-60 positive rate) included in the main cell population developed in FSC-SSC dot plot was analyzed. Results are shown in Tables 1 to 3 and Fig. 5. Fig. 5 shows results of Examples 1 and 2 and Comparative Examples 1 and 2 and the number N. In Fig. 5, data of P2 and P4 are shown in order from the left. As shown in Tables 1 to 3 and Fig. 5, in Examples 1 to 3, a cell population (cell population including iPS cells) having a high positive rate of TRA-1-60 was obtained in a short period of time.

Details and results are shown in Tables 1 to 3 below and drawings. In Example 4, establishment of iPS cells was visually confirmed.

**[Table 1]**

| | | Example 1 | Example 2 |
|---|---|---|---|
| Type of somatic cells | | Peripheral blood mononuclear cells | Peripheral blood mononuclear cells |
| Cell culture substrate | Type | Culture container (P) | Culture container (L) |
| | Cell scaffold | PVB-AA | Laminin |
| Seeding step | Seeding amount (cells/well) | 8.0 × 10^4 | 8.0 × 10^4 |
| | Seeding density (cells/cm^2) | 2.5 × 10^5 | 2.5 × 10^5 |
| Removal step | | Removing suspended cells | Removing suspended cells |
| Recovery step | | - | - |
| Introducing step | Cells into which reprogramming factor has been introduced | Cells adhering to cell scaffold | Cells adhering to cell scaffold |
| | MOI | 0.75 | 0.75 |
| | Residual rate of virus (GFP positive rate (average)) | 0.38% (P2) | 0.83% (P2) |
| Evaluation | | 0.05% (P3) | 0.06% (P3) |
| | Undifferentiation property (TRA-1-60 positive rate) | 90.5% (P2) | 88.9%(P2) |
| | | 90.3% (P4) | 89.2%(P4) |

**[Table 2]**

| | | Example 3 | Example 4 |
|---|---|---|---|
| Type of somatic cells | | Monocytes | Peripheral blood mononuclear cells |
| Cell culture substrate | Type | Culture container (P) | Microcarrier (P) |
| | Cell scaffold | PVB-AA | PVB-AA |
| Seeding step | Seeding amount (cells/well) | 2.0×10^4 | 1.6×10^5 |
| | Seeding density (cells/cm^2) | 6.3×10^4 | 5.5×10^4 |
| Removal step | | Removing suspended cells | Removing suspended cells |
| Recovery step | | - | - |
| Introducing step | Cells into which reprogramming factor has been introduced | Cells adhering to cell scaffold | Cells adhering to cell scaffold |
| | MOI | 0.75 | 0.75 |
| Evaluation | Residual rate of virus (GFP positive rate (average)) | 0.03% (P2) | - |
| | | 0.03% (P3) | |
| | Undifferentiation property (TRA-1-60 positive rate) | 61.9% (P2) | - |
| | | 76.0% (P4) | |

**[Table 3]**

| | | Comparative Example 1 | Comparative Example 2 |
|---|---|---|---|
| Type of somatic cells | | Peripheral blood mononuclear cells | Peripheral blood mononuclear cells |
| Cell culture substrate | Type | Culture container (P) | Culture container (P) |
| | Cell scaffold | PVB-AA | PVB-AA |
| Seeding step | Seeding amount (cells/well) | 2.0×10^6 | 2.0×10^6 |
| | Seeding density (cells/cm^2) | 6.3×10^6 | 6.3×10^6 |
| Removal step | | - | - |

| Recovery step | | Recovering adhering cells | Recovering suspended cells |
|---|---|---|---|
| Introducing step | Cells into which reprogramming factor has been introduced | Recovered cells | Recovered cells |
| | MOI | 3 | 3 |
| Evaluation | Residual rate of virus (GFP positive rate (average)) | 0.15% (P2) | 1.31% (P2) |
| | | 0.07% (P3) | 0.85% (P3) |
| | Undifferentiation property (TRA-1-60 positive rate) | 77.1%(P2) | 76.2% (P2) |
| | | 75.1% (P4) | 75.6% (P4) |

Fig. 6 is a micrograph of a colony of iPS cells established from monocytes in Example 3. More specifically, Fig. 6 is a micrograph (magnification: 100 times) on Day 5 after the cells of P2 are cultured.

## Claims

1. A production method for induced pluripotent stem cells, comprising, in the stated order:
a seeding step of seeding somatic cells on a cell culture substrate including a cell scaffold;
a removal step of removing suspended somatic cells without adhering to the cell scaffold; and
an introducing step of introducing a reprogramming factor into somatic cells adhering to the cell scaffold.

2. The production method for induced pluripotent stem cells according to claim 1, wherein the somatic cells seeded in the seeding step include peripheral blood mononuclear cells.

3. The production method for induced pluripotent stem cells according to claim 1 or 2, wherein the somatic cells adhering to the cell scaffold in the introducing step include cells having phagocytosis.

4. The production method for induced pluripotent stem cells according to any one of claims 1 to 3, wherein the cell scaffold has an RGD sequence.

5. The production method for induced pluripotent stem cells according to any one of claims 1 to 4, wherein the cell scaffold contains a peptide-conjugated resin having a synthetic resin moiety and a peptide moiety.

6. The production method for induced pluripotent stem cells according to claim 5, wherein the peptide-conjugated resin has a polyvinyl acetal resin moiety and a peptide moiety.

7. The production method for induced pluripotent stem cells according to any one of claims 1 to 6, wherein in the introducing step, a reprogramming factor is introduced into somatic cells adhering to the cell scaffold using a sendaiviral vector.
